# EUROPEAN PATENT APPLICATION

(11) **EP 4 697 339 A1**
(43) Date of publication of application: **18.02.2026**
(21) Application number: 24196870.0
(22) Date of filing: 28.08.2024
(51) Int. Cl.: G16H 15/00, G16H 40/63

(54) **CONTROLLING DISPLAY**

(30) Priority: 14.08.2024 US 202463682951 P
(71) Applicant: Koninklijke Philips N.V., 5656 AG Eindhoven (NL)
(72) Inventor: HUIJBREGTS, Laurentia Johanna, 5656 AG Eindhoven (NL); KUHLMANN, Daan, 5656 AG Eindhoven (NL); VAN LIESHOUT, Ron Martinus Laurentius, 5656 AG Eindhoven (NL)
(74) Representative: Philips Intellectual Property & Standards

(57) **Abstract**

Proposed concepts thus aim to provide schemes, solutions, concepts, designs, methods and systems pertaining to controlling a display. In particular, embodiments aim to provide a method for controlling a display by generating an information signal which describes many physiological parameters and then generating a display control signal which only displays a subset of the physiological parameters included in the information signal (e.g., which are deemed to be of more clinical importance than the others).

## Description

### FIELD OF THE INVENTION

This invention relates to the field of controlling a display.

### BACKGROUND OF THE INVENTION

Avatars have recently begun being used on medical displays to provide visual representations of a subject's vital signs (and/or other relevant clinical parameters) through, for example, a virtual model of the monitored subject, indicating the healthiness of their vital signs through color, shape and/or animation. In this way, an avatar can provide an overview of a subject's health state at a quick glance. An avatar typically has a set of parameter thresholds for individual clinical parameters, and the visualization of said clinical parameters typically depends on the configuration of this parameter set. For example, if the measured values exceed or fall below a threshold, an animation can represent this transition accordingly.

However, for displays visualizing one or more avatars and/or other forms of physiological/clinical parameters, there may be too much information provided for a clinician to make sense of in an efficient manner. In other words, modern medical displays which often indicate many different physiological parameters (using, for example, patient avatars, trendlines, numerics, etc.) can sometimes provide information overload.

### SUMMARY OF THE INVENTION

The invention is defined by the claims.

According to examples in accordance with an aspect of the invention, there is provided a computer-implemented method for controlling a display.

The method comprises: generating an information signal describing a plurality of physiological parameters of at least one subject and a form of display for each of the plurality of physiological parameters; determining a total quantity-of-information score describing an amount of information described by the information signal; determining a priority subset and a non-priority subset of the plurality of physiological parameters based on the total quantity-of-information score, wherein the priority and non-priority subsets are mutually exclusive; and generating a display control signal for controlling a display, defining the display of the priority subset and the non-display of the non-priority subset.

Proposed concepts thus aim to provide schemes, solutions, concepts, designs, methods and systems pertaining to controlling a display. In particular, embodiments aim to provide a method for controlling a display by generating an information signal which describes many physiological parameters (and how they would be displayed) and then generates a display control signal which only displays a subset of the physiological parameters (e.g., which are deemed to be of more clinical importance than the others).

In other words, it is proposed that after an information signal has been generated describing a plurality of physiological parameters (of one or more subjects/patients), a score can be worked out for the signal indicating how much information the information signal contains, and therefore would cause a display unit to display if all the parameters were to be displayed. Based on how high this score is, a display control signal can then be accordingly generated to essentially hide a proportion of the information. In a simple example, if the information signal does not describe very much data, then all of the information can be displayed via the generated display control signal, whereas if it describes quite a lot of data, then a few pieces of information can be kept from being included in the display control signal, and if it describes a massive amount of data, then most of the information can be kept from being included in the display control signal.

For example, this method of controlling a display could be utilized to control a bedside medical display, a central display (e.g., for overseeing a number of subjects in a ward, for example), a VR/AR/MR headset, or any other suitable display, as would be understood by the skilled person.

In summary, the method provides a way to reduce/simplify the display of a large amount of potentially complicated data so that less information is displayed and therefore can be more easily understood, thus allowing a clinician to more efficiently spend their time. For example, this can facilitate the response time of a clinician being reduced, thereby leading to earlier interventions and thus fewer complications. Furthermore, this can help focus a clinician's attention on the right patient's parameter(s), thereby leading to more useful interventions and thus fewer complications.

In some embodiments, determining the priority subset and non-priority subset may be based on at least one aspect of each of the plurality of physiological parameters. For example, this may allow the physiological parameters to be hierarchically ordered (i.e., prioritized), so that the less important/useful physiological parameters can be put in the non-priority subset and thus not displayed (i.e., hidden).

In some embodiments, an aspect may comprise at least one of: a percentage change; an absolute change; a predetermined range for the specific physiological parameter; a rate of change; a direction of change; and whether the physiological parameter is a past, present, or predicted future physiological parameter. These may all be useful aspects of physiological parameters to take into account when hierarchically ordering them in terms of clinical importance / usefulness.

In some embodiments, the method may further comprise: generating a second information signal describing a second plurality of physiological parameters of the at least one subject at a later time; determining a second total quantity-of-information score describing an amount of information described by the second information signal; determining a second priority subset and a second non-priority subset of the second plurality of physiological parameters based on the total quantity-of-information score and the second total quantity-of-information score, wherein the second priority and second non-priority subsets are mutually exclusive; and generating a second display control signal for controlling the same display, defining the display of the second priority subset and the non-display of the second non-priority subset. For example, this may allow for a change and/or rate of change of the total quantity-of-information score over time to be taken into account when determining the priority and non-priority subsets. For instance, if the total quantity-of-information score rapidly increases (e.g., new physiological parameters start to be monitored), the priority and non-priority subsets may be gradually adjusted to slowly add (some of or all) the extra information over time so as to reduce sudden information overload.

In some embodiments, the information signal may describe at least one of: a first avatar of a first subject, the first avatar visually indicating at least one of the plurality of physiological parameters; a trendline of at least one of the plurality of physiological parameters; a waveform of at least one of the plurality of physiological parameters; and a numeric of at least one of the plurality of physiological parameters. These may all be suitable forms of a physiological parameter's display.

In some embodiments, the method may further comprise determining an individual quantity-of-information score for each of the plurality of physiological parameters based on the type of physiological parameter and/or the form of the physiological parameter's display; and wherein determining the total quantity-of-information score comprises summing the individual quantity-of-information scores of the plurality of physiological parameters. Both the type of physiological parameter (i.e., what parameter it actually is) and the form of the physiological parameter (e.g., displayed in an avatar, using a trendline, using a waveform, using a numeric, etc.) can affect the cognitive load needed to understand the information and may therefore be advantageously taken into account when calculating the total quantity-of-information score.

In some embodiments, determining the non-priority subset may comprise determining a subset of the plurality of physiological parameters with a sum of associated individual quantity-of-information scores which is equal to or greater than the value by which the total quantity-of-information score exceeds a predetermined upper threshold. For example, this may provide an effective way to determine how many physiological parameters need to be non-displayed / hidden in order to sufficiently reduce the displayed information to a preferred level.

In some embodiments, the predetermined upper threshold may be adjustable by a user. This may thus allow a user to set a preferable maximum amount of information to be displayed at any one time, as different users may have different preferences / tolerances for this.

In some embodiments, a total quantity-of-information score of the display control signal may be above a predetermined lower threshold. In this way, it may be ensured that not too much information is hidden and enough information is still displayed to a clinician.

In some embodiments, the method may further comprise adjusting the display control signal, responsive to user input, to temporarily display the non-priority subset. This may allow a user to still temporarily view (at least part of) the non-priority subset if they wish to see it, thus providing a way for a user to still be provided with more information but only when they are ready to see it (i.e., specifically request to see it).

In some embodiments, the non-priority subset may automatically not be displayed again after a predetermined time period. In this way, a user need not manually hide the non-priority subset again after they have viewed it.

In some embodiments, the predetermined time period may be responsive to at least one of: a total quantity-of-information score of the non-priority subset; a measurement frequency of at least one of the physiological parameters of the non-priority subset; and/or a type of physiological parameter of at least one of the physiological parameters of the non-priority subset. These may all be useful elements to take into account during the determination of when to automatically re-hide the temporarily displayed non-priority subset.

According to another aspect of the invention, there is provided a computer program comprising code means for implementing the method of any herein disclosed method when said program is run on a processing system.

According to another aspect of the invention, there is provided a system for controlling a display. The system comprising a display; and a processor configured to: generate an information signal describing a plurality of physiological parameters of at least one subject and a form of display for each of the plurality of physiological parameters; determine a total quantity-of-information score describing an amount of information described by the information signal; determine a priority subset and a non-priority subset of the plurality of physiological parameters based on the total quantity-of-information score, wherein the priority and non-priority subsets are mutually exclusive; and generate a display control signal for controlling the display, defining the display of the priority subset and the non-display of the non-priority subset.

In some embodiments, determining the priority subset and non-priority subset may be based on at least one aspect of each of the plurality of physiological parameters.

Thus, there may be proposed concepts for controlling a display, and this may be done based on generating an information signal which describes two or more physiological parameters and then generating a display control signal which only shows a portion of the information.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a simplified flow diagram of a method for controlling a display according to a proposed embodiment;
Fig. 2 is a flow diagram of a method for controlling a display according to a proposed embodiment;
Fig. 3 is a flow diagram of a method for controlling a display according to a proposed embodiment;
Fig. 4 is a simplified block diagram of a system for controlling a display according to a proposed embodiment; and
Fig. 5 illustrates an example of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Implementations in accordance with the present disclosure relate to various techniques, methods, schemes and/or solutions pertaining to controlling a display. According to proposed concepts, a number of possible solutions may be implemented separately or jointly. That is, although these possible solutions may be described below separately, two or more of these possible solutions may be implemented in one combination or another.

Embodiments of the invention aim to provide a method for controlling a display. This can be achieved by generating an information signal which describes many physiological parameters (and how they would be displayed) and then generates a display control signal which only displays a subset of the physiological parameters (e.g., which are deemed to be of more clinical importance than the others).

In other words, it is proposed that after an information signal has been generated describing a plurality of physiological parameters (of one or more subjects/patients), a score can be worked out for the signal indicating how much information the information signal contains, and therefore would cause a display unit to display if all the parameters were to be displayed. Based on how high this score is, a display control signal can then be accordingly generated to essentially hide a proportion of the information. In a simple example, if the information signal does not describe very much data, then all of the information can be displayed via the generated display control signal, whereas if it describes quite a lot of data, then a few pieces of information can be kept from being included in the display control signal, and if it describes a massive amount of data, then most of the information can be kept from being included in the display control signal.

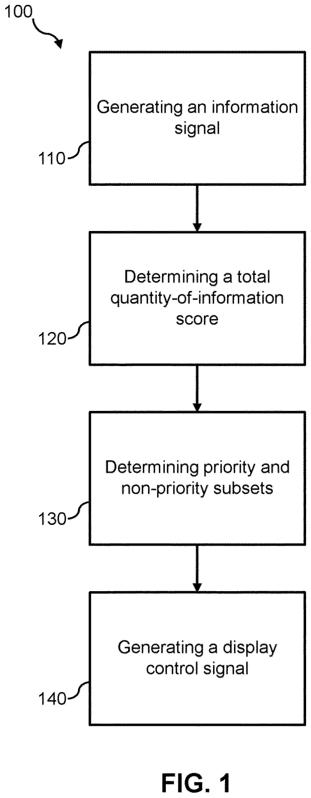

Referring now to Fig. 1, there is depicted a simplified flow diagram of a method 100 for controlling a display according to a proposed embodiment.

The method 100 comprises step 110 of generating an information signal describing a plurality of physiological (i.e., clinical) parameters of at least one subject and a form of display for each of the plurality of physiological parameters. For example, the information signal can be understood as a signal which describes two or more physiological parameters and also describes how they would be displayed on a display (i.e., the form of their display, e.g., avatar, trendline, numeric, etc.). In other words, the signal describes/contains all the parameters which could potentially be displayed (and how they would be displayed, i.e., their form) but does not specify where they would be displayed on a display unit, i.e., is not necessarily a display control signal.

For instance, in this embodiment, a physiological (i.e., clinical) parameter comprises at least one of: SpO2; heart rate; pulse rate; respiration rate; ST elevation; blood pressure; central venous pressure; end-tidal CO2; body temperature; cardiac output; cardiac index; brain activity; relaxation status; FiO2; airway pressure; tidal volume; hypotension prediction; hemodynamic instability prediction; sepsis prediction; and hypoxia prediction. These are all useful parameters for a clinician to be aware of, but of course, in other embodiments, any suitable physiological/clinical parameter could be used, as would be understood by the skilled person.

In this embodiment, the information signal describes at least one of: a first avatar of a first subject, the first avatar visually indicating at least one of the plurality of physiological parameters; a trendline of at least one of the plurality of physiological parameters; a waveform of at least one of the plurality of physiological parameters; and a numeric of at least one of the plurality of physiological parameters. In other words, the form of display for each of the plurality of physiological parameters described by the information signal comprises at least one of the above list. These are all be suitable forms of a physiological parameter's display, but in other embodiments, the information signal can describe any suitable form of display of each of the physiological parameters, as would be understood by the skilled person.

For example, an avatar can visually indicate physiological parameter(s) using at least one of: an icon (e.g., of an organ or of a snowflake to indicate low body temperature, for example); a color (e.g., red to indicate a parameter outside of a normal range); a border (e.g., thick or thin to indicate high or low blood pressure respectively); and an animation (e.g., a heart pumping quickly to indicate a high heart bpm). In this embodiment, an avatar comprises a graphical representation of a living being. This is a particularly effective form of avatar for indicating physiological parameters. Specifically in this embodiment, however, the living being comprises a person (i.e., the avatar comprises a graphical representation of a person). This, again, is a particularly effective form of avatar for indicating physiological parameters for people. In other embodiments, however, the living being can be an animal (e.g., for when the subject is an animal, or perhaps for children to pick for themselves).

For example, a trendline can be understood as a graphical representation of changes in a physiological parameter over time. For example, a waveform can be understood as a graphical representation of a physiological signal or measurement over time. For example, a numeric can be understood as one or more numerical values representing a physiological parameter (e.g., at a given point in time).

Step 120 comprises determining a total quantity-of-information score describing an amount of information described by the information signal. In other words, step 120 comprises calculating a score that represents the total amount of information that would be displayed if all the physiological parameters described by the information signal were displayed on a display unit (in the forms specified by the information signal). In other words, step 120 comprises calculating a score that serves as a proxy for the cognitive load needed to understand the information contained within the information signal. In yet other words, the information signal is analyzed in order to determine how much information it is describing, and a numerical score is then determined to represent this amount. The skilled person would appreciate that this could be performed in a variety of suitable ways.

Step 130 comprises determining a priority subset and a non-priority subset of the plurality of physiological parameters based on (at least) the total quantity-of-information score, wherein the priority and non-priority subsets are mutually exclusive (i.e., each physiological parameter can be put in either the priority subset or the non-priority subset, but not both). For example, if the total quantity-of-information score is high, one or more of the physiological parameters can be put in the non-priority - for instance, this could be done randomly (i.e., randomly picking one or more of the physiological parameters), or it could be done in a last-in, first-out basis (i.e., the physiological parameters described last by the information signal are the first to be put into the non-priority subset), or it could be done via any suitable hierarchy system. The skilled person would understand that there are many ways in which the priority and non-priority subsets could be determined. It should be noted that in some embodiments, the non-priority subset can, of course, be empty, i.e., all the physiological parameters can be determined to be in the priority subset, however, in no situation would all of the physiological parameters be determined to be in the non-priority subset.

In this embodiment, however, determining the priority subset and non-priority subset is based on at least one aspect of each of the plurality of physiological parameters. For example, this allows the physiological parameters to be hierarchically ordered (i.e., prioritized), so that the less clinically important/useful physiological parameters can be put in the non-priority subset and thus not displayed (i.e., hidden) via the display control signal. As the skilled person would understand, there are many aspects of physiological parameters which could be used in order to help determine the clinical importance of each physiological parameter (i.e., in order to determine which should go in the priority subset and which in the non-priority subset), however, in this embodiment, an aspect specifically comprises at least one of: a percentage change; an absolute change; a predetermined range for the specific physiological parameter; a rate of change; a direction of change (e.g., towards or away from an ideal value for the specific physiological parameter); and whether the physiological parameter is a past, present, or predicted future physiological parameter. These are all useful aspects of physiological parameters to take into account when hierarchically ordering them in terms of clinical importance / usefulness.

These provided aspects can also be grouped into certain concepts (and in some embodiments, the aspects can be restricted to one or more of these groups). For instance, a first group of aspects relates to changes: a percentage change; an absolute change; a rate of change; a direction of change. A second group of aspects relates to thresholds and/or ranges: a predetermined range for the specific physiological parameter (e.g., if a parameter is outside a goal/target range or inside the goal/target range). It should also be noted that there can be multiple predetermined ranges for a specific physiological parameter, such as a normal/target/expected range, a yellow alarm range, a red alarm range, a goal range, a custom range, etc. A third and final group of aspects relates to whether the physiological parameter is a past (i.e., historic), present, or future physiological parameter (predicted by an algorithm, for example).

It should be noted, that the change and rate of change aspects can be understood as a change or rate of change within a certain time period (e.g., the past 1 minute, 5 minutes, 10 minutes, 30 minutes, one hour, two hours, etc.), and this time period could vary depending on the type of physiological parameter (e.g., for parameters which can change rapidly, such as heart rate, the time window can be smaller than for parameters which cannot change rapidly, such as a hormone level).

It should also be noted that in embodiments wherein the information signal describes a patient avatar (which uses predetermined ranges to determine how to visually indicate each physiological parameter), it is efficient (and thus preferred) to use the same ranges as an aspect of the physiological parameters.

Finally, step 140 comprises generating a display control signal for controlling a display, defining the display of the priority subset and the non-display (i.e., the hiding/concealment) of the non-priority subset. For example, the information signal could thus be understood as a *potential-*full-view display control signal and the display control signal could thus be understood to as a simplified-view display control signal.

The display can comprise any suitable display, e.g., a medical display, a monitor, a smart phone screen, a tablet screen, or the display of a VR/AR/MR headset, etc., as the skilled person would understand.

As the skilled person would understand, there are many ways in which this could be done, for example, in some embodiments, the display control signal could contain no information regarding the physiological parameters in the non-priority subset at all, whereas in other embodiments, the display control signal could still contain the information regarding the physiological parameters in the non-priority subset but also specify that the non-priority subset should be hidden/concealed from view (e.g., by making it totally transparent or positioning it off the screen or overlaying an opaque image/color, or any other suitable method, as would be understood by the skilled person).

Further, in this embodiment, a total quantity-of-information score of the display control signal is above a predetermined lower threshold (i.e., the priority and non-priority subsets are determined such that the total quantity-of-information score of the priority subset is above a predetermined lower threshold). In this way, it can be ensured that not too much information is hidden and enough information is still displayed.

In a simple example, the information signal describes a patient avatar showing respiration rate (normal), heart rate (normal), and body temperature (too low). Based on the total quantity-of-information, it is determined to only put the body temperature in the priority subset (as it is abnormal while the other parameters are normal), and so a display control signal is generated which only defines the display of a patient avatar indicating body temperature, whereas the indications of the respiration rate and heart rate indications are not displayed/hidden.

It should be noted that in some embodiments, the information signal may, in fact, comprise a display control signal for controlling the display, the display control signal defining the display of all of the plurality of physiological parameters of the at least one subject. In this case, the method 100 can thus be alternatively understood as: generating a display control signal for controlling a display, the display control signal defining the display of a plurality of physiological parameters of at least one subject; determining a total quantity-of-information score describing an amount of information to-be-displayed defined by the display control signal; determining a priority subset and a non-priority subset of the plurality of physiological parameters based on the total quantity-of-information score, wherein the priority and non-priority subsets are mutually exclusive; and adjusting the display control signal to generate a modified display control signal defining the display of the priority subset and the non-display of the non-priority subset.

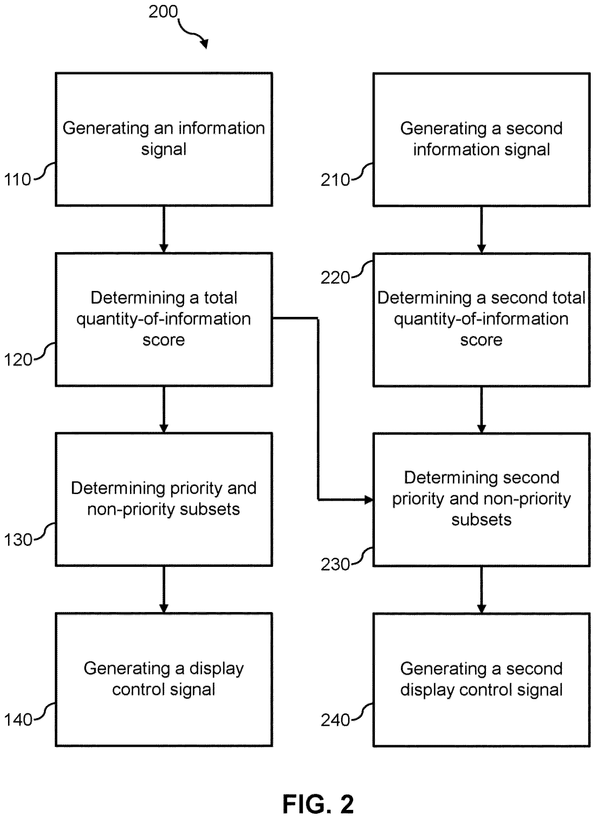

Referring now to Fig. 2, there is depicted a flow diagram of a method 200 for controlling a display according to a proposed embodiment. Steps 110, 120, 130 and 140 are substantially the same as have been described in relation to method 100 of Fig. 1.

Step 210 comprises generating a second information signal describing a second plurality of physiological parameters of the at least one subject at a later time (i.e., after the generation of the display control signal in step 140). For example, step 210 should take place after the display control signal of step 140 has been output to a display unit.

Step 220 comprises determining a second total quantity-of-information score describing an amount of information described by the second information signal.

Step 230 comprises determining a second priority subset and a second non-priority subset of the second plurality of physiological parameters based on the total quantity-of-information score (determined in step 120) and the second total quantity-of-information score (determined in step 220), wherein the second priority and second non-priority subsets are mutually exclusive.

Step 240 then comprises generating a second display control signal for controlling the same display (unit), defining the display of the second priority subset and the non-display of the second non-priority subset.

In other words, this allows for an absolute/percentage change or rate of change of the total quantity-of-information score over time to be taken into account when determining the priority and non-priority subsets. For example, if the quantity-of-information score determined in step 120 was 10 (arbitrary) and the second quantity-of-information score (determined in step 220) is now 20 (due to the addition of more physiological parameters), as this is a doubling of information (i.e. a large change), the second display control signal can be adapted to only add some of the new information to the priority subset (such that a large amount of new information is not simultaneously provided to a clinician). This can thus reduce the cognitive load on the clinician. Furthermore, when a patient's situation is changing quickly, the rate at which new display control signals (e.g., second display control signals) are generated can also be increased in order to more adaptively respond to these changes.

In another example, rather than the number of physiological parameters changing between the first and second quantity-of-information scores, the values of the parameters could have changed, thus changing the importance of said parameters which would justify a change of priority and non-priority subsets. In another example, the threshold for the maximum quantity-of-information score may have changed in the meantime, thus justifying a change of priority and non-priority subsets.

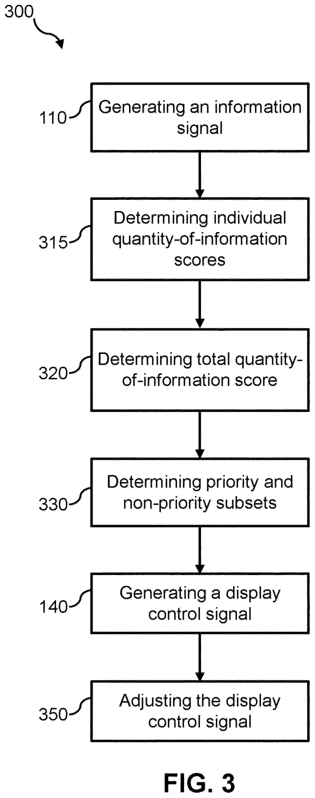

Referring now to Fig. 3, there is depicted a method 300 for controlling a display according to a proposed embodiment. Steps 110 and 140 are substantially the same as the method 100 of Fig. 1.

Step 315 comprises determining an individual quantity-of-information score for each of the plurality of physiological parameters based on the type of physiological parameter and/or the form of the physiological parameter's display. To be clear, the type of physiological parameter can be understood as what the parameter actually is, e.g., heart rate or respiration rate or haemoglobin level or body temperature or sepsis prediction, etc. The form of the physiological parameter's display is instead *how* the parameter is presented, e.g., displayed as part of an avatar, or displayed as a trendline, or displayed as a waveform, or displayed a numeric, etc. Each physiological parameter of the plurality of physiological parameters thus has an individual quantity-of-information score which can take into account either of or (preferably) both what it is and how it is presented. For example, a heart rate numeric may have a score of one, whereas a heart rate waveform may have a score of three, whereas a respiration rate animation in an avatar may have a score of two. In another example, where only the form of physiological parameter is taken into account, a numeric could always add 2 points, regardless of what the physiological parameter actually is, and in another example, where only the type of physiological parameter is taken into account, a heart rate could always add 2 points, regardless of how the physiological parameter is presented (i.e., its form).

Determining the total quantity-of-information score in step 320 thus comprises summing the individual quantity-of-information scores of the plurality of physiological parameters. Both the type of physiological parameter (i.e., what parameter it actually is) and the form of the physiological parameter (e.g., displayed in an avatar, using a trendline, using a waveform, using a numeric, etc.) can both affect the cognitive load needed to understand the information and may therefore be advantageously taken into account when calculating the total quantity-of-information score. It should be noted that in other embodiments, step 320 can comprise combining the individual quantity-of-information scores in other ways, for example, inputting them into an algorithm and/or using some form of weighting or multiplication for some combinations of information.

Step 330 is substantially the same as step 130 of method 100, however, in this embodiment, determining the non-priority subset comprises determining a subset of the plurality of physiological parameters with a sum of associated individual quantity-of-information scores which is equal to or greater than the value by which the total quantity-of-information score exceeds a predetermined upper threshold. For example, this provides an effective way to determine how many physiological parameters need to be not displayed / hidden in order to sufficiently reduce the displayed information to a preferred level. For example, this predetermined upper threshold can be manually set or automatically determined (e.g., dependent on context). It should thus be noted that if the total quantity-of-information score does not exceed the predetermined upper threshold, then no physiological parameters need to be put in the non-priority subset.

In this embodiment, the predetermined upper threshold is also adjustable by a user. This thus allows a user to set a preferable maximum amount of information to be displayed at any one time, as different users may have different preferences / tolerances for this.

Step 350 comprises adjusting the display control signal (generated in step 140 and after it has been output to a display unit (step not shown)), responsive to user input, to temporarily display (i.e., unhide / un-conceal) (at least one of the physiological parameters of) the non-priority subset. This can thus allow a user to still temporarily view (at least a portion of) the non-priority subset if they wish to see it, thus providing a way for a user to still be provided with more information but only when they are ready to see it, i.e., specifically request to see it. For example, in this embodiment, the user input can comprise at least one of: using an input device (e.g., a mouse or touch screen, etc.); gaze control; voice control; and gesture control. In other embodiments, however, any suitable form of user input can be used, as would be understood by the skilled person.

In this embodiment, the non-priority subset can be automatically hidden (i.e., concealed or not displayed again) after a predetermined time period. For example, the predetermined time period can be manually set or automatically set. In this way, a user need not manually hide the non-priority subset again after they have viewed it. For example, the predetermined time period could be thirty seconds, or one minute, or five minutes, or any other suitable time period.

In this embodiment, the predetermined time period is responsive to at least one of: a total quantity-of-information score of (at least one of the physiological parameters of the) the non-priority subset (which have been temporarily displayed); a measurement frequency of at least one of the physiological parameters of the non-priority subset; and/or a type of physiological parameter of at least one of the physiological parameters of the non-priority subset. These can all be useful elements to take into account during the determination of when to automatically re-hide the temporarily displayed non-priority subset. For example, if the total quantity-of-information score of the non-priority subset (or one or more physiological parameters of the non-priority subset which are being temporarily displayed) is high, then the predetermined time period can be longer, as the information is likely to take longer for a clinician to understand.

The measurement frequency can be understood as the frequency with which a physiological parameter is measured, i.e., having its values updated. As described above, the type of physiological parameter can be understood as what the parameter actually is, e.g., heart rate or respiration rate or haemoglobin level or body temperature or sepsis prediction, etc. This is because what the parameter actually is informs how fast it is possible for it to physically change. For example, certain parameters physically cannot change quickly, such as hormone levels like thyroid hormones T4 and T3, or heart wall thickness - in these cases, it will take at least an hour before there is a non-negligible change. However, other parameters can change very quickly, such as heart rate. Therefore, parameters which are likely to stay constant for a long time can be hidden more quickly than parameters which are likely to / able to rapidly change, and which would therefore be of more interest for a clinician to visually monitor, if they desire.

In a simple example of the invention, the total quantity-of-information score indicates how much information would be on the screen (if all the parameters described by the information signal were to be displayed according to each of their described forms of display respectively). In particular, the invention first counts how much information would be on the screen without any simplifications. A full patient avatar with all its parameters would, for example, add 25 points to the total quantity-of-information score. Another full patient avatar would add another 25 points. Then, if the information signal also describes three waveforms, which add 5 points each, the total quantity-of-information score would be 65. Assuming a predetermined upper threshold is set to 50, some of the information would thus have to be hidden. It is thus determined to use a simplified view for one of the patient avatars (i.e., to put some of its physiological parameters in the non-priority subset). This can reduce the information from that avatar to 10 points, which would thereby bring the total quantity-of-information score of the generated display control signal to the predetermined upper threshold of 50. When there is a clear preference for one of the two avatars to show in full (for example, when the other avatar is only referring to a past or predicted future state of the same patient), then the current avatar would ideally be kept in full view and the other one (past or future) would be simplified. If there is no clear preference for one of the two avatars to remain in full view, then they could both be simplified in order not to create confusion. It is okay for the total quantity-of-information score to drop substantially below the predetermined upper threshold as long as it does not go below a predetermined lower threshold (if one has been set).

The points a visual patient avatar will add to the total quantity-of-information score can depend on the number of physiological/clinical parameters it indicates. For example, if there are only a few types of measurements (e.g. heart rate, blood pressure, and core body temperature, while no other measurements are performed on this patient), then the avatar may add only 10 points to the total quantity-of-information score instead of 25 points for a visual patient avatar with all kinds of measurements. In such a case, both visual patient avatars and the three waveforms will together not exceed the threshold of 50 and thus both visual patient avatars can be shown in full view (along with the waveforms).

In a straightforward embodiment, the total quantity-of-information score has only one threshold; if the value is above this threshold, the information on the screen should be simplified (i.e., some parameters hidden), while (at/)below the threshold it does not need to be simplified. In other embodiments, there may be more thresholds. There may be a threshold below which the system would strive to add more information on the screen. The rules for this selection can depend on the total quantity-of-information score. For example, when the information amount variable is below the lower threshold in a simplified view that only shows variables that are out of range, but on the other hand the total quantity-of-information score would get above the higher threshold if the full visual patients were shown, the system can choose the extra rules to, in the simplified view, also show parameters that are changing fast.

It should be noted that adding more visual patient avatars to the screen can result in the visual patient avatars being depicted smaller than when only a few visual patients are on the screen, because of the available screen size. However, the size of an avatar is in this invention is not relevant, but rather simplification is guided by the actual amount of information on the screen, i.e., not the size of it. Nevertheless, avatars can also get reduced in size when they are simplified, i.e. size and simplification may show some correlation, but size is not the causal effect. An avatar can stay the same size and be simplified at one moment (when there is a lot of other information on the screen) and depicted fully (i.e., with all its parameters) at another moment.

As stated above, it is also possible for a user to manually switch to a full view of an avatar (i.e., showing all of its parameters). For example, this can be done with manual user interactions such as tapping on the screen on the avatar or clicking with a mouse on the avatar, or with other user interactions such as eye gaze or gesture control. In response to such a user interaction, the screen can change such that the maximum threshold of the total quantity-of-information score is (temporarily) exceeded. In this case, the system could switch back to its old view after e.g. 1 minute, to make sure that the total quantity-of-information score is again acceptable. In another response to the user interaction, the system could show the full view of the avatar that was selected by the user and at the same time reduce (i.e., hide) other information on the screen (by e.g. making simplified views of the other avatar(s) on the screen or removing trendlines/waveforms). In this way, the total quantity-of-information score might stay below the predetermined upper threshold, and the view would not need to switch back. In other words, this can be understood as a way for a user to indicate a preference as to which parameters are in the priority subset.

In some embodiments, the method/system can even learn from user interactions, such as when a user wants to see a full view of an avatar. For the same user, or a caregiver in the same role (nurse, physician, ...) in similar situations (with respect to phase in the workflow, patient situation, etc.), the system could essentially learn when to show the full view of the avatar (i.e., when to have all of an avatar's physiological parameters put in the priority subset).

While the above can be seen as a temporary exceedance of the information amount variable threshold, in some embodiments, the user can also be in control of the predetermined upper threshold/limit for the total quantity-of-information score. For example, the user could enter a value, or use a mouse wheel or other rotary kind of control, to finetune the total quantity-of-information score threshold.

In another example, there are three visual patient avatars described by the information signal. These belong to three different patients and there is no clear preference to show one of them in more detail than the others. In this example, every physiological parameter depicted in the patient avatar adds one point to the total quantity-of-information score. In full view of an avatar, all measured parameters are shown. For patient 1 there are 12, for patient 2 there are 10 and for patient 3 there are 13 measured parameters. Thus, if all avatars were shown in full view, the total quantity-of-information score would be 35. Suppose further that the (upper) total quantity-of-information score threshold is 30. Therefore, showing all three avatars in full would not be allowed. Suppose further that for patient 1 there are 8 parameters in normal range and not changing fast, and for patients 2 and 3 there are respectively 7 and 8 of such parameters. Therefore, simplifying all avatars by leaving out (from the display control signal) all parameters that are in normal range and not changing fast, will bring the total quantity-of-information score to 12 points. This is far below the threshold. Therefore, there is room to show something more. Suppose further that all three patients each have one parameter that is in the normal range and not changing fast, but nevertheless is very close to an alarm limit (i.e., a range threshold). Then the system could be set up to show those parameters as well. So, in this case, the range boundaries, given by the alarm limits, are not hard thresholds for whether or not to show the parameter, but the distance to the alarm limit can also be taken into account. Besides, in this example, it is not chosen to have one or two avatar(s) in full view, while the other one or two are in simplified view (where parameters in normal range that don't change fast are omitted), but all three avatars have an in-between visualization.

In the above examples, embodiments with patient avatars were mainly described, however, as described, the concepts can be applied to other forms of presenting physiological parameters. For example, trendlines of physiological parameters (i.e., their values over time) can be shown in addition to or instead of avatars. Trendlines cannot be simplified in the same way that an avatar can be (by removing visualizations of parameters) so they can either be binarily shown or hidden.

In yet another example of the invention, there can be a pre-defined order of what types of physiological parameter should be simplified/hidden first (when the total quantity-of-information score is too high). The simplification order could comprise:
1.) An avatar indicating past physiological parameters
2.) An avatar indicating predicted future physiological parameters
3.) Trendlines
4.) An avatar indicating current physiological parameters
5.) Waveforms
6.) Numerics

For example, if the information signal only describes a current avatar and several trendlines (and the total quantity-of-information score was too high), then the trendlines could be hidden first (i.e., not included in the generated display control signal) before any consideration is made of simplifying the current avatar.

In some embodiments, the determination of which physiological parameters to put in the non-priority subset can further depend on the potential decrease in the total quantity-of-information score. For example, if removing the trendlines decreases the total quantity-of-information score by 5 points and this is not enough to get below the upper threshold, but a current avatar could be simplified and this would decrease the total quantity-of-information score by 15 points (thereby getting below the upper threshold), then the current avatar could be simplified and the trendlines left in the priority subset, even though the trendlines were higher on the simplification order list. In some embodiments, the simplification order list is not a pre-defined list that is the same regardless of context, but rather it could depend on the particular user (e.g., clinician or caregiver), their role, the patient status, and/or phase in the workflow, etc.

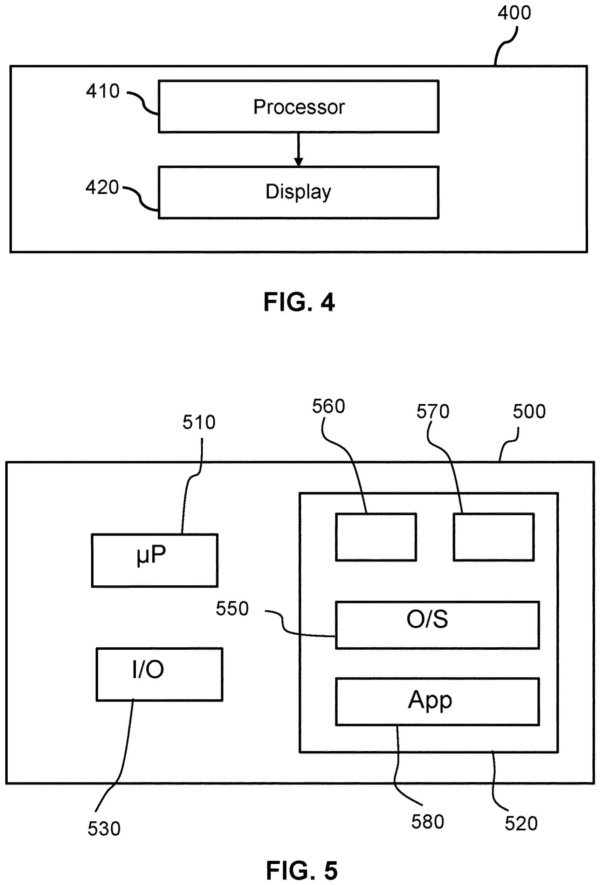

Referring now to Fig. 4, there is depicted a system 400 for controlling a display according to a proposed embodiment. The system 400 comprises a processor 410 and a display 420.

The display 420 can comprise at least one of: a monitor; a virtual reality (VR) headset; an augmented reality (AR) headset; a projector; a television screen; a laptop screen; a tablet display; a smartphone display; an e-ink display; a heads-up display (HUD); a smart mirror; a holographic display; a wearable display, such as smart glasses; an LED matrix display; a flexible OLED display; and any other suitable display, as would be understood by the skilled person.

The processor 410 is configured to: generate an information signal describing a plurality of physiological parameters of at least one subject and a form of display for each of the plurality of physiological parameters; determine a total quantity-of-information score describing an amount of information described by the information signal; determine a priority subset and a non-priority subset of the plurality of physiological parameters based on the total quantity-of-information score, wherein the priority and non-priority subsets are mutually exclusive; and generate a display control signal for controlling the display 420, defining the display of the priority subset and the non-display of the non-priority subset.

In this embodiment, determining the priority subset and non-priority subset are based on at least one aspect of each of the plurality of physiological parameters.

Fig. 5 illustrates an example of a computer 500 within which one or more parts of an embodiment may be employed. Various operations discussed above may utilize the capabilities of the computer 500. In this regard, it is to be understood that system functional blocks can run on a single computer or may be distributed over several computers and locations (e.g. connected via internet).

The computer 500 includes, but is not limited to, PCs, workstations, laptops, PDAs, palm devices, servers, storages, and the like. Generally, in terms of hardware architecture, the computer 500 may include one or more processors 510, memory 520 and one or more I/O devices 530 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 510 is a hardware device for executing software that can be stored in the memory 520. The processor 510 can be virtually any custom made or commercially available processor, a central processing unit (CPU), a digital signal processor (DSP), or an auxiliary processor among several processors associated with the computer 500, and the processor 510 may be a semiconductor based microprocessor (in the form of a microchip) or a microprocessor.

The memory 520 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 520 may incorporate electronic, magnetic, optical, and/or other types of storage media. Note that the memory 520 can have a distributed architecture, where various components are situated remote from one another, but can be accessed by the processor 510.

The software in the memory 520 may include one or more separate programs, each of which comprises an ordered listing of executable instructions for implementing logical functions. The software in the memory 520 includes a suitable operating system (O/S) 550, compiler 560, source code 570, and one or more applications 580 in accordance with exemplary embodiments. As illustrated, the application 580 comprises numerous functional components for implementing the features and operations of the exemplary embodiments. The application 580 of the computer 500 may represent various applications, computational units, logic, functional units, processes, operations, virtual entities, and/or modules in accordance with exemplary embodiments, but the application 580 is not meant to be a limitation.

The operating system 550 controls the execution of other computer programs, and provides scheduling, input-output control, file and data management, memory management, and communication control and related services. It is contemplated by the inventors that the application 580 for implementing exemplary embodiments may be applicable on all commercially available operating systems.

Application 580 may be a source program, executable program (object code), script, or any other entity comprising a set of instructions to be performed. When a source program, then the program is usually translated via a compiler (such as the compiler 560), assembler, interpreter, or the like, which may or may not be included within the memory 520, so as to operate properly in connection with the O/S 550. Furthermore, the application 580 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 530 may include input devices such as, for example but not limited to, a mouse, keyboard, scanner, microphone, camera, etc. Furthermore, the I/O devices 530 may also include output devices, for example but not limited to a printer, display, etc. Finally, the I/O devices 530 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 530 also include components for communicating over various networks, such as the Internet or intranet.

If the computer 500 is a PC, workstation, intelligent device or the like, the software in the memory 520 may further include a basic input output system (BIOS) (omitted for simplicity). The BIOS is a set of essential software routines that initialize and test hardware at start-up, start the O/S 550, and support the transfer of data among the hardware devices. The BIOS is stored in some type of read-only-memory, such as ROM, PROM, EPROM, EEPROM or the like, so that the BIOS can be executed when the computer 500 is activated.

When the computer 500 is in operation, the processor 510 is configured to execute software stored within the memory 520, to communicate data to and from the memory 520, and to generally control operations of the computer 500 pursuant to the software. The application 580 and the O/S 550 are read, in whole or in part, by the processor 510, perhaps buffered within the processor 510, and then executed.

When the application 580 is implemented in software it should be noted that the application 580 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 580 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods of Figs. 1-3, and the system of Fig. 4, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Fig. 5 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions, the architecture, functionality, and operation of possible implementations of systems, methods, and computer program products according to various embodiments of the present invention.

## Claims

1. A computer-implemented method (100) for controlling a display, the method comprising:
generating an information signal (110) describing a plurality of physiological parameters of at least one subject and a form of display for each of the plurality of physiological parameters;
determining a total quantity-of-information score (120) describing an amount of information described by the information signal;
determining a priority subset and a non-priority subset (130) of the plurality of physiological parameters based on the total quantity-of-information score, wherein the priority and non-priority subsets are mutually exclusive; and
generating a display control signal (140) for controlling a display, defining the display of the priority subset and the non-display of the non-priority subset.

2. The computer-implemented method of claim 1, wherein determining the priority subset and non-priority subset is based on at least one aspect of each of the plurality of physiological parameters.

3. The computer-implemented method of claim 2, wherein an aspect comprises at least one of: a percentage change; an absolute change; a predetermined range for the specific physiological parameter; a rate of change; a direction of change; and whether the physiological parameter is a past, present, or predicted future physiological parameter.

4. The computer-implemented method of any of claims 1 to 3, wherein the method further comprises:
generating a second information signal (210) describing a second plurality of physiological parameters of the at least one subject at a later time and a form of display for each of the second plurality of physiological parameters;
determining a second total quantity-of-information score (220) describing an amount of information described by the second information signal;
determining a second priority subset and a second non-priority subset (230) of the second plurality of physiological parameters based on the total quantity-of-information score and the second total quantity-of-information score, wherein the second priority and second non-priority subsets are mutually exclusive; and
generating a second display control signal (240) for controlling the same display, defining the display of the second priority subset and the non-display of the second non-priority subset.

5. The computer-implemented method of any of claims 1 to 4, wherein the information signal describes at least one of: a first avatar of a first subject, the first avatar visually indicating at least one of the plurality of physiological parameters; a trendline of at least one of the plurality of physiological parameters; a waveform of at least one of the plurality of physiological parameters; and a numeric of at least one of the plurality of physiological parameters.

6. The computer-implemented method of any of claims 1 to 5, wherein the method further comprises:
determining an individual quantity-of-information score (315) for each of the plurality of physiological parameters based on the type of physiological parameter and/or the form of the physiological parameter's display; and wherein determining the total quantity-of-information score comprises summing the individual quantity-of-information scores (320) of the plurality of physiological parameters.

7. The computer-implemented method of claim 6, wherein determining the non-priority subset comprises determining a subset of the plurality of physiological parameters (330) with a sum of associated individual quantity-of-information scores which is equal to or greater than the value by which the total quantity-of-information score exceeds a predetermined upper threshold.

8. The computer-implemented method of claim 7, wherein the predetermined upper threshold is adjustable by a user.

9. The computer-implemented method of any of claims 1 to 8, wherein a total quantity-of-information score of the display control signal is above a predetermined lower threshold.

10. The computer-implemented method of any of claims 1 to 9, wherein the method further comprises: adjusting the display control signal (350), responsive to user input, to temporarily display the non-priority subset.

11. The computer-implemented method of claim 10, wherein the non-priority subset is automatically not displayed again after a predetermined time period.

12. The computer-implemented method of claim 11, wherein the predetermined time period is responsive to at least one of: a total quantity-of-information score of the non-priority subset; a measurement frequency of at least one of the physiological parameters of the non-priority subset; and/or a type of physiological parameter of at least one of the physiological parameters of the non-priority subset.

13. A computer program product comprising computer program code means which, when executed on a computing device having a processing system, cause the processing system to perform all of the steps of the computer-implemented method according to any of claims 1 to 12.

14. A system (400) for controlling a display, the system comprising:
a display (420); and
a processor (410) configured to:
generate an information signal describing a plurality of physiological parameters of at least one subject and a form of display for each of the plurality of physiological parameters;
determine a total quantity-of-information score describing an amount of information described by the information signal;
determine a priority subset and a non-priority subset of the plurality of physiological parameters based on the total quantity-of-information score, wherein the priority and non-priority subsets are mutually exclusive; and
generate a display control signal for controlling the display, defining the display of the priority subset and the non-display of the non-priority subset.

15. The system of claim 14, wherein determining the priority subset and non-priority subset is based on at least one aspect of each of the plurality of physiological parameters.
